(19) **Europäisches Patentamt**
**European Patent Office**
**Office européen des brevets**

(11) **EP 4 603 014 A1**

(12) **EUROPEAN PATENT APPLICATION**

(43) Date of publication:
**20.08.2025 Bulletin 2025/34**

(21) Application number: **24157451.6**

(22) Date of filing: **13.02.2024**

(51) International Patent Classification (IPC):
*A61B 5/113* (2006.01)     *A61B 5/11* (2006.01)

(52) Cooperative Patent Classification (CPC):
**A61B 5/1135; A61B 5/1127; A61B 5/1128**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Max-Planck-Gesellschaft zur
Förderung der
Wissenschaften e.V.
80539 München (DE)**

(72) Inventors:
• **Svetlova, Anzhelika**
  **37070 Göttingen (DE)**
• **Alves, Prof. Dr. Frauke**
  **37070 Göttingen (DE)**
• **Dullin, Prof. Dr. Christian**
  **37070 Göttingen (DE)**

(74) Representative: **WSL Patentanwälte
Partnerschaft mbB
Kaiser-Friedrich-Ring 98
65185 Wiesbaden (DE)**

(54) **OPTICAL LUNG FUNCTION MEASUREMENT**

(57) A method and apparatus for determining a chest function of a subject are provided. The method includes projecting, by a projector, a projection pattern including a number of optical markers onto a chest of the subject. The method further comprises capturing, by a stereo-camera system, a series of images of the projection pattern from at least two angles as the subject breathes. Each optical marker from the projection pattern is imaged from both of the at least two angles at the same time. The method further comprises tracking, by a processor, a movement over time of the projection pattern based on the captured series of images by tracking a movement over time of each of the optical markers in the captured series of images. The method also comprises determining, by the processor, at least one parameter indicative of the chest function of the subject based on a result of the tracking.

FIG. 1

EP 4 603 014 A1

**Description**

**TECHNICAL FIELD**

The present invention relates to a method and apparatus for determining a chest function of a subject. In particular, the present invention relates to a method and apparatus for measuring a lung function of the subject.

**BACKGROUND**

Due to their significant burden on public health, lung diseases are a key focus of preclinical and clinical research efforts globally. The most important symptom of lung disease is impaired lung function. However, obtaining reliable lung function measurements can be difficult, especially in small animals and non-compliant subjects.

It is in this context that the present invention is derived.

**BRIEF SUMMARY**

In one aspect, a method for determining a chest function of a subject is provided. The method includes projecting, by a projector, a projection pattern including a number of optical markers onto a chest of the subject. The method further comprises capturing, by a stereo-camera system, a series of images of the projection pattern from at least two angles as the subject breathes. Each optical marker from the projection pattern is imaged from both of the at least two angles at the same time. The method further comprises tracking, by a processor, a movement over time of the projection pattern based on the captured series of images by tracking a movement over time of each of the optical markers in the captured series of images. The method also comprises determining, by the processor, at least one parameter indicative of the chest function of the subject based on a result of the tracking.

Advantageously, this is a non-invasive method of determining a chest function of a subject. The subject does not need to be awake or compliant, and the method can be used in the same way for both animal and human subjects.

Further advantageously, projecting the optical markers onto the chest of the patient means that the patient does not need to be touched or manipulated. The optical markers can be more accurately positioned compared to physical markers. Furthermore, optical markers are less likely to undergo unwanted movement (such as slipping) as the subject breathes, so the measurements obtained using optical markers are more reliable.

Further advantageously, the data obtained using this method has a high temporal resolution and different breathing cycles are not averaged. It is therefore possible to more accurately derive a chest function of the subject.

Further advantageously, this method obtains accurate and reproduceable results without requiring dedicated specialist hardware.

Moreover, when determining the chest function of the subject, the breathing motion of the subject causes each of the optical markers to be modulated in space. Each of these modulated optical markers can be tracked across time in every camera view. This enables a more accurate reconstruction of the motion of the chest to be achieved, and so the at least one parameter indicative of the chest function can be more reliably derived.

Optionally, the method further comprises generating a unique identifier (ID) for each optical marker.

Advantageously, generating a unique ID for at least some and preferably most or even each optical marker means that it is easier to track the thus specified markers from both angles as the patient breathes. The generated ID is consistent between all camera views and represents the same optical marker in space. This ensures that the stereo-camera system has a highly accurate starting point for these optical markers, so the tracking of the optical markers is improved.

Moreover, since the ID of the optical markers is consistent between camera views, it is possible to link the optical marker of the same ID from all filmed directions. This ensures robustness against the variability in lighting, camera aperture and aids in situations of bad focus while maintaining high spatial resolution. This therefore improves the tracking of the optical markers and the subsequent reconstruction of the chest movement.

Optionally, generating the unique ID comprises projecting, by the projector, a series of optical marker arrangements onto the chest of the subject. Each arrangement includes one or more of the optical markers from the projection pattern, and each optical marker is present in at least one arrangement from the series of arrangements. The method further comprises detecting, by the stereo-camera system, the one or more optical markers in each of the projected arrangements from the at least two angles. The method also comprises generating, by the processor, the ID for each optical marker based on the at least one arrangement in which the optical marker is detected.

Advantageously, the unique ID for each optical marker can be obtained while the subject is breathing, and therefore while the optical markers are moving. It is not necessary for the subject to hold their breath so the subject does not need to be compliant or conscious. Furthermore, it is not possible to perform breath-hold manoeuvres in animals without invasive tracheotomy procedures, so this method allows the unique IDs to be obtained in a non-invasive manner.

Optionally, tracking the movement over time of the projection pattern comprises generating one or more of a two-dimensional (2D) projection and a three-dimensional (3D) projection of the projection pattern at each of a series of time points. The method further comprises extracting, from the one or more generated projections, a movement over time of each of the optical markers of the projection pattern.

Optionally, determining the at least one parameter indicative of the chest function of the subject may comprise reconstructing a four-dimensional (4D) motion of the chest based on a result of the tracking of the projection pattern. The method may further include extracting, from the reconstructed motion of the chest, the at least one parameter.

Optionally, the at least one parameter comprises one or more of a general temporal parameter, a local temporal parameter, a moving speed of a portion of the chest, a diaphragm motion parameter, a tidal volume indicator, or a parameter indicative of a lung function of the subject.

Optionally the general temporal parameter or local temporal parameter are selected from the group consisting of: inspiration, expiration times, speed of inspiration, speed of fast expiration, area under the curve of slow expiration, decay of expiration, baseline (as air trapping), flow.

Advantageously, these parameters are good indicators of the functioning of the chest and/or the lungs of the subject.

Optionally, the sampling rate is at least 10 frames per second.

Optionally, the sampling rate is less than 200 frames per second.

Optionally, one or more of a size of each of the optical markers, a spacing between each of the optical markers, and a number of the optical markers is determined based on a size of a surface area the of chest of the subject.

Due to the significant size differences between humans and mice, and the very different speeds at which their chests move, it is difficult to measure the chest function of a mouse and a chest function of a human using the same method. Advantageously, the present invention enables the array to be customised according to the subject being monitored so the same method may be used regardless of the subject. This method can therefore be used for both mice and human subjects. In on example the subject may be a humans or an animals. The terms "subject", "individual", and "patient" are used herein interchangeably. Examples of subjects include mammals, e.g., humans, dogs, cows, horses, pigs, sheep, goats, cats, mice, rabbits, rats, guinea pigs, hamsters, non-human primates, and transgenic non-human animals. The subject may be an infant. In some examples, the subject is a human. The subject may be a human infant. The term "human infant" as used herein is intended to refer to a child in the first year of life.

Further advantageously, using the same method for both human and mice subjects improves the translation of data between humans and mice. That is, the data obtained for both human and mice subjects can be more easily compared. This is important because mice are frequently used as the subjects in pre-clinical trials. However, clinical tests are more frequently performed on human subjects. When different methods are used to monitor the chest functions of mice and humans, the results cannot be directly compared. By providing a chest function measurement method which is usable for both mice and humans, preclinical and clinical test results can be more easily compared. This can lead to improved outcomes for patients undergoing clinical procedures.

Optionally, the optical markers are unequally distributed across the chest such that regions of the chest which have a greater range of movement while the subject is breathing and/or are more indicative of the chest function of the subject have a greater density of optical markers.

Advantageously, this allows more precise reconstructions of the chest in areas which are of high importance for determining the chest function of the subject. Areas of the chest which are more indicative of a chest function of the subject may have a higher density of markers than areas of the chest which are less significant. This enables the tracking to focus on the more important areas of the chest in greater detail, and therefore improves the accuracy and reliability of the chest function measurement.

Further advantageously, the movement of the chest in regions which undergo relatively high levels of movement during expiration may be more precisely reconstructed. The more precise reconstruction of the chest movement also enables the accuracy and reliability of the chest function measurement to be improved.

Optionally, the optical markers have a luminance of at least twice a background luminance.

Advantageously, relatively bright markers are more visible to the stereocameras so it is easier for the cameras to distinguish and track each of the markers. Markers having more luminance are also less susceptible to noise which provides more accurate measurements.

In another aspect, an apparatus for determining a chest function of a subject is provided. The apparatus comprises a projector configured to project a projection pattern of optical markers onto a chest of the subject. The apparatus also includes stereo-camera system configured to capture a series of images of the projection pattern from at least two angles as the subject breathes, such that each optical marker from the projection pattern is imaged from both of the at least two angles at the same time. The apparatus also includes a processor. The processor is configured to track a movement over time of each of the optical markers based on the captured series of images, and determine at least one parameter relating to the chest function of the subject based on a result of the tracking.

Optionally, the processor is further configured to control the projector to project the projection pattern of optical markers onto the chest of the subject. The processor is also configured to control the stereo-camera system to capture the series of images of the projection pattern as the subject breathes.

Optionally, the apparatus further comprises a mounting structure for mounting to a holding means for holding the subject.

Optionally, the apparatus also comprises the holding means for holding the subject. The holding means comprises a support structure for supporting a body of the subject and an anaesthesia mask for providing anaesthesia to the subject.

Existing preclinical respiratory function tests compromise precision and proximity to physiological condition with unrestrained whole body plethysmography being closer to natural physiological state and ventilation tracheotomy having higher precision. A new measurement method Optical lung function (OLF) based on the optical computer vision principles is described herein. OLF has the following advantages: a) it is non-invasive, easy and fast to perform, b) it is close to the physiological breathing conditions and c) it is readily compatible with longitudinal measurements. Lung function parameters obtained by our novel OLF approach allow differentiation between two lung disease mouse models of allergic airway inflammation and pulmonary fibrosis and healthy controls at the acute stage of the disease.

## BRIEF DESCRIPTION OF THE SEVERAL VIEWS OF THE DRAWINGS

To easily identify the discussion of any particular element or act, the most significant digit or digits in a reference number refer to the figure number in which that element is first introduced.

FIG. 1 shows a schematic illustration of an apparatus for determining a chest function of a subject according to an embodiment of the invention.

FIG. 2 shows a schematic illustration of an apparatus for determining a chest function of a subject according to a further embodiment of the invention

FIG. 3 is a flow chart showing a method for determining a chest function of a subject according to an embodiment of the invention.

FIG. 4 is a flow chart showing a method for determining a chest function of a subject according to a further embodiment of the invention

FIG. 5 illustrates a sequence of encoding patterns according to an embodiment.

FIG. 6 illustrates a 2D projection of a mesh according to an embodiment.

FIG. 7 illustrates a 3D projection of a mesh according to an embodiment.

FIG. 8 is a series of graphs illustrating a movement over time of a point of a mesh according to an embodiment.

FIG. 9 shows an example of a mounting structure and a holding means according to an embodiment.

FIG. 10 shows an example of displacement curves from different anatomical regions.

FIG. 11 shows the corresponding anatomical regions for the graphs of FIG. 10.

FIG, 12 shows a segmentation of an abdominal placement curve.

FIG. 13 shows a comparison of abdominal placement curves between animal subjects.

FIG. 14 shows a comparison of the displacement meshes derived by μCT and OLF for control mice and a mouse model with Severe acute allergic asthma (SAA) and Pulmonary Fibrosis (PF).

FIG. 15 shows several lung parameters measured by means of OLF, μCT and XLF.

## DETAILED DESCRIPTION

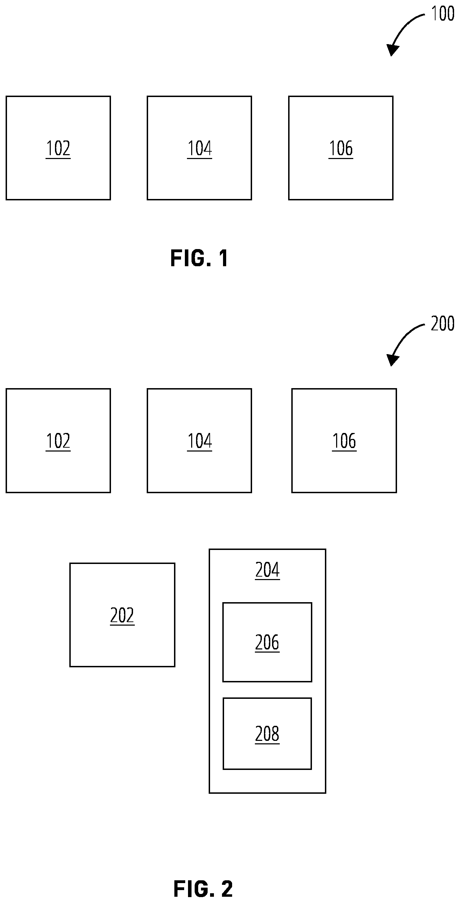

FIG. 1 shows an example of an apparatus 100 for measuring a chest function of a subject, such as a human or a mouse. The apparatus 100 comprises a projector 102, a stereo-camera system 104, and a processor 106.

The projector 102 is configured to project a projection pattern onto the chest of the subject. The projection pattern comprises a plurality of optical markers. The optical markers function as artificial landmarks which are trackable by an optical detection system, such as the stereo-camera system 104. The optical markers may be arranged in a structured pattern, such as a grid or an array. The optical markers may use light of any frequency or wavelength which can be detected by the stereo-camera system 104. In particular, the light may have a wavelength which falls within the visible spectrum. Using light within the visible spectrum advantageously improves the ease of use of the apparatus 100 for the human operator. Visible light optical markers are also more likely to be compatible with standard commercially available camera systems, eliminating the need for costly and specialised equipment. Where the optical markers use visible light, the luminance of the optical markers may be at least twice that of the background luminance. The background luminance is the luminance of the environment surrounding the subject on which the optical markers are projected.

The stereo-camera system 104 is an optical detection system for detecting and tracking the movement of each of the projected optical markers as the subject breathes. The stereo-camera system 104 is configured to capture a series of images of the projection pattern from at least two angles as the subject breathes. The stereo-camera system comprises at least two camera units, wherein each camera unit comprises a lens and an optical sensor, which is usually a two-dimensional array of sensor pixels. Each camera unit generates two-dimensional images of the subject. Even each point of the subject is viewed by the at least two camera units with at least two angles the optical axis of the camera units can be parallel to each other. The stereo-camera system 104 may comprise a single camera which forms two camera units and which is able to capture images at two distant sections of the optical sensor simultaneously, so that any point of the subject is viewed from two different angles.

Each of the optical markers in the projection pattern is imaged from both of the at least two angles at the same time.

The stereo-camera system may be configured to capture the series of images at a sampling rate of at least 10 frames per second, at least 20 frames per second, at least 30 frames per second, at least 40 frames per second, or at least 50 frames per second.

The stereo-camera system may be configured to capture the series of images at a sampling rate of less than 100 frames per second, less than 150 frames per second, less than 200 frames per second, less than 250 frames per second, or less than 500 frames per second.

The sampling rate of the stereo-camera system as it captures the series of images may be a number between any combination of the aforementioned sampling rates.

The processor 106 is configured to determine at least one parameter indicative of the chest function of the subject based on a result of the tracking performed by the stereo-camera system 104. The processor 106 may be further configured to control the projector to project the projection pattern of optical markers onto the chest of the subject. The processor 106 may also be configured to control the stereo-camera system 104 to capture the series of images of the projection pattern as the subject breathes.

FIG. 2 shows another example of an apparatus 200 for measuring a chest function of a subject. The apparatus 200 comprises the projector 102, stereo-camera system 104 and processor 106 described in relation to FIG. 1.

The apparatus 200 further comprises a mounting structure 202 for mounting to a holding means 204 for holding the subject. Advantageously, the holding means 204 allows the subject to be held without directly touching the subject. The mounting structure 202 and holding means 204 together enable the subject to be positioned and manoeuvred more precisely and without directly touching the subject.

The holding means 204 may comprise a support structure 206 for supporting or holding the body of the subject. The holding means 204 may further comprise an anaesthesia mask 208 for providing anaesthesia to the subject.

The holding means 204 and mounting structure 202 may be particularly useful when the apparatus 200 is being used to measure a chest function of an animal, such as a mouse. The reduced need for direct human contact with the animal may reduce any distress to the animal whilst still allowing the position of the animal to be precisely controlled.

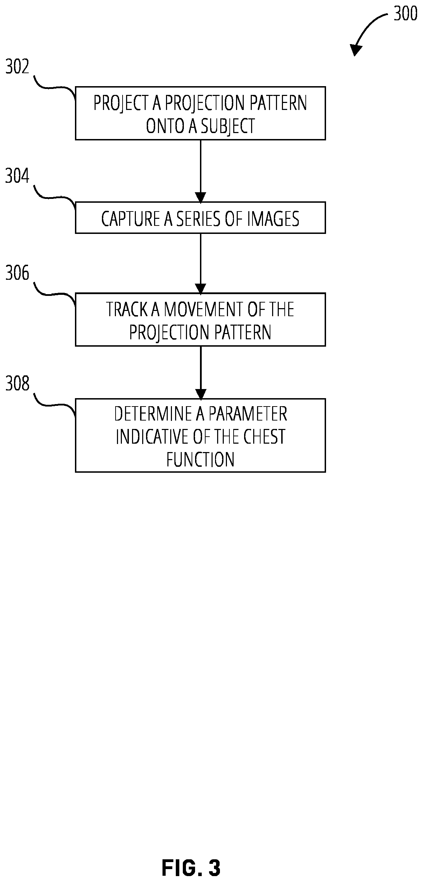

FIG. 3 shows an example of a method for measuring a chest function of a subject. The method comprises projecting, 302, a projection pattern comprising a number of optical markers onto the chest of the subject.

One or more of a size of each of the optical markers, a spacing between each of the optical markers, and a number of the optical markers may be determined based on a size of a surface area the of chest of the subject. For example, the optical markers used for a subject having a chest with a relatively small surface area (such as a mouse) may have a smaller diameter than the diameter of the optical markers used for a subject having a chest with a relatively large surface area (such as a human). As another example, the optical markers used for a mouse subject may be more closely spaced together than the optical markers used for a human subject. The skilled person would recognise that the properties of the optical markers could be adapted in any number of ways based on the size of the surface area of the chest, and are not limited to these examples.

The optical markers in the projection pattern may be unequally distributed across the chest of the patient. For example, regions of the chest which have a greater range of movement while the subject is breathing and/or are more indicative of the chest function of the subject may have a greater density of optical markers than regions which undergo less movement or have less significance on the determination of the chest function.

The method further comprises capturing, 304, a series of images of the projection pattern from at least two angles as the subject breathes. Each optical marker from the projection pattern is imaged from both of the at least two angles at the same time.

The method further comprises tracking, 306, a movement over time of the projection pattern based on the captured series of images. This is performed by tracking a movement over time of each of the optical markers in the captured series of images.

Tracking the movement of the projection pattern over time may be performed by generating one or more of a 2D projection and a 3D projection of the projection pattern at a plurality of time points from the series of time points. The at least one of the 2D and 3D projection may be generated from each of the series of time points. The movement over time of each of the optical markers of the projection pattern may then be extracted from the one or more of the generated 2D and 3D projection patterns.

The method further comprises determining, 308, at least one parameter indicative of the chest function of the subject based on a result of the tracking.

Determining the at least one parameter may comprise reconstructing a 4D motion of the chest based on a result of tracking the movement of the projection pattern. The at least one parameter may then be extracted from the 4D reconstruction of the chest motion.

The at least one parameter may comprise one or more of a general temporal parameter, a local temporal parameter, a moving speed of a portion of the chest, a diaphragm motion parameter, a tidal volume indicator, or a parameter indicative of a lung function of the subject.

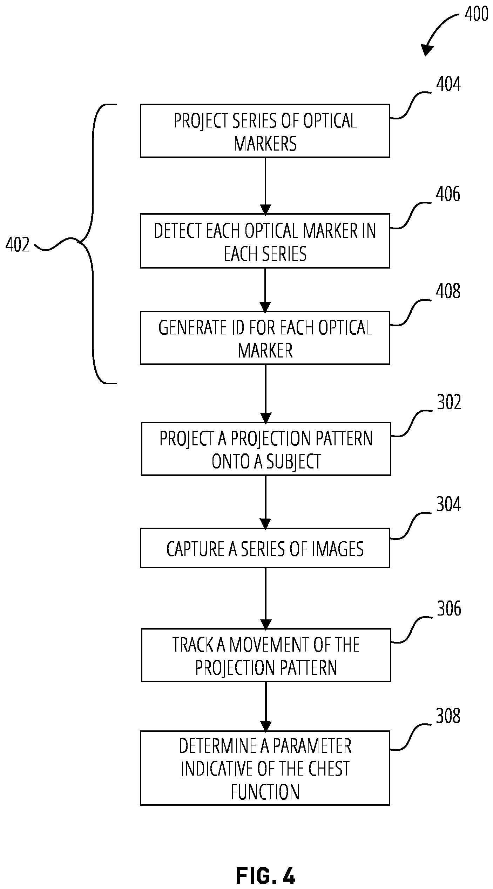

FIG. 4 shows another example of a method 400 for measuring a chest function of a subject according to another embodiment of the invention. As well as steps 302, 304, 306, and 308 described in relation to FIG. 3, the method further comprises generating, 402, a unique identifier (ID) for each optical marker. The step of generating 402 the ID for each optical marker may be referred to as the encoding phase. The unique IDs are generated before the projection pattern is projected onto the chest of the subject in step 302.

Generating, 402, the unique ID comprises projecting, 404, a series of optical marker arrangements onto the chest of the subject. Each of the optical marker arrangements comprises one or more of the optical markers from the projection pattern. Each optical marker from the projection pattern is present in at least one arrangement from the series of optical marker arrangements.

Generating, 402, the unique ID further comprises detecting 406 the one or more optical markers in each of the projected arrangements from two or more different angles.

Generating 402 the unique ID further comprises generating 408 the ID for at least some, preferably most or even each optical marker based on the at least one arrangement in which the optical marker is detected.

In more detail, in the encoding phase, the optical markers may be projected in a blast sequence of pre-composed images. These precomposed images are projected one after another with a digital projector. These precomposed images are digital images stored in a control unit and these images comprises small patterns which form the markers and which are always located in the same position in the image to form the same marker. Each marker can be coded by a specific sequence of patterns which are all projected to the same place/direction. The coding can be carried out by changing the colour, size, form, level of light intensity of the marker, and any combination of these properties. One preferred example is are blinking signals for each coded marker, where the blinking signals can be distinguished by frequency, light intensity or pulse length.

The number of markers may be at least 10, 20, 50, 100, 250, or 500.

This produces an easily detectable unique ID for each optical marker which can be recognised by any of the cameras of the stereo-camera system 104. The total number (N) of optical marker arrangements (also known as encoding patterns) to generate the unique IDs may be calculated as $N = \log_2(E) + 1$, where E is the number of required optical markers (also known as elements) in the projection pattern used for determining the chest function parameter.

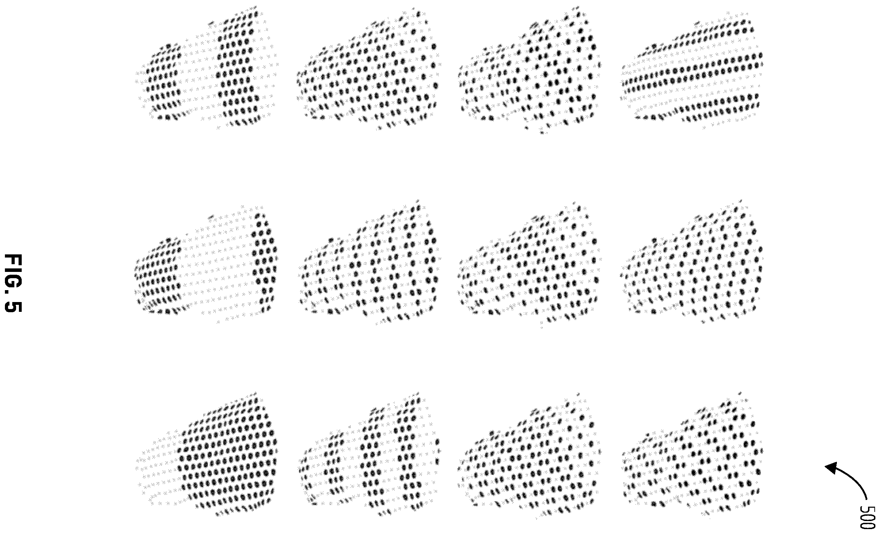

The size of the elements is restricted to the projector and camera resolutions. The number of elements can be modified according to need. For a small animal a dense grid is preferred due to the animal size. For the application to a human patient, a larger grid may be sufficient. The grid spacing is inversely proportional to spatial resolution of the final reconstruction and to the corresponding computation time. During the encoding, each element, while remaining static in space, gets temporal information across all projected image by turning the element signal (S) "on" or "off". Figure 5 shows an example of a sequence of 12 images which are to be projected onto the subject one after another during an encoding phase. Each of the images shows a number of markers. In this example the markers are digitally coded by the dark dots as "on" and the light dots as "off". The elements get detected in each projected image and an ID is generated by the sum of values from multiplying the signal (0 for "off" and 1 for "on") with the binary multiplier $m = 2^n$ where n is the projected image number $n = \in [0, N]$ like so:

$$ID = \sum_{n=0}^{N}\left(S_n * m\right)$$

An example of a single derived ID for a single marker can be seen in Table 1. To identify the markers, certain positions are monitored in the sequence of images and it is checked whether these positions show an "on" or "off" signal. The sequence of "on" and "off" values is detected and forms the sequence S in the following table.

Table 1: Example of single element decoding.

| n | 0 | 1 | 2 | 3 | 4 | 5 | 6 | 7 | 8 | 9 | 10 | 11 | 12 |
|---|---|---|---|---|---|---|---|---|---|---|----|----|----|
| m | 1 | 2 | 4 | 8 | 16 | 32 | 64 | 128 | 256 | 512 | 1024 | 2048 | 4096 |
| S | 1 | 0 | 0 | 0 | 0 | 1 | 0 | 1 | 1 | 1 | 0 | 0 | 0 |
| Decoded | 1 | 0 | 0 | 0 | 0 | 32 | 0 | 128 | 256 | 512 | 0 | 0 | 0 |
| ID | 929 | | | | | | | | | | | | |

Such a calculation is carried out for each position of interest in the images or marker in the images, respectively, to identify the corresponding ID.

For measuring a chest function, all projected markers or only a certain, predefined, selection of markers can be used.

The generated ID is consistent between all camera views and represents the same element in space.

In certain cases breath-hold manoeuvres are not possible or counter indicated in a patient. Furthermore, it is not possible to do such a manoeuvre in an animal without invasive tracheotomy. Therefore the encoding ensures that the stereo-camera has a highly accurate starting point for each element which is not disturbed by the continuous breathing motion of the subject, does not require a compliant subject, and does not require invasive procedures performed on the subject.

The encoding phase is followed by a dynamic phase where a grid with all elements as "on" is projected on the breathing subject. This grid is the projection pattern previously described. The dynamic phase is the phase in which the steps of method 300 are performed.

In the dynamic phase each of the elements is modulated in space due to breathing motion. Each of these modulated elements is tracked across time in every camera view. Since the ID of each element is consistent between camera views, it is possible to link the elements of the same ID from all filmed directions. This ensures robustness against the variability in lighting, camera aperture and aids in situations of bad focus while maintaining high spatial resolution, therefore making the tracking and reconstruction easier compared to the prior art. Based on the disparity principle of the stereo-camera, the 4D motion of the outer chest may then reconstructed. It is then possible to derive several relevant functional parameters from the various locations of the dynamic surface.

FIG. 5 shows an example of a series of encoding patterns used to generate an ID according to step 402. The encoding patterns are projected onto a chest of the subject in a series of short blasts, from which a unique ID for each optical marker can be generated. The encoding patterns are then followed by a projection pattern, which may be a static image, used for determining the at least one parameter relating to the chest function of the subject.

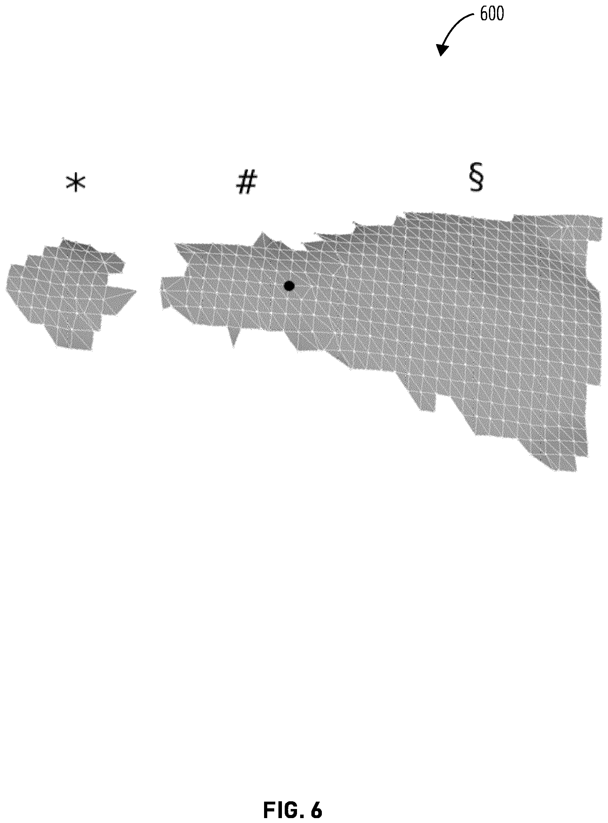

FIG. 6 shows an example of a 2D projection of a generated mesh at one time point. The 2D projection displays the neck (*), chest (9) and abdomen (§) of the subject. Due to the encoding to generate the unique IDs for each optical marker, the generation of the mesh is relatively simple. Optical markers that cannot reliably be identified in both cameras may be automatically removed from this process so that they are not used to generate the 2D (or 3D) projection. This ensures that only the optical markers which have been reliably tracked are used for the 2D or 3D projections, which improves the accuracy and reliability of the determined parameter result.

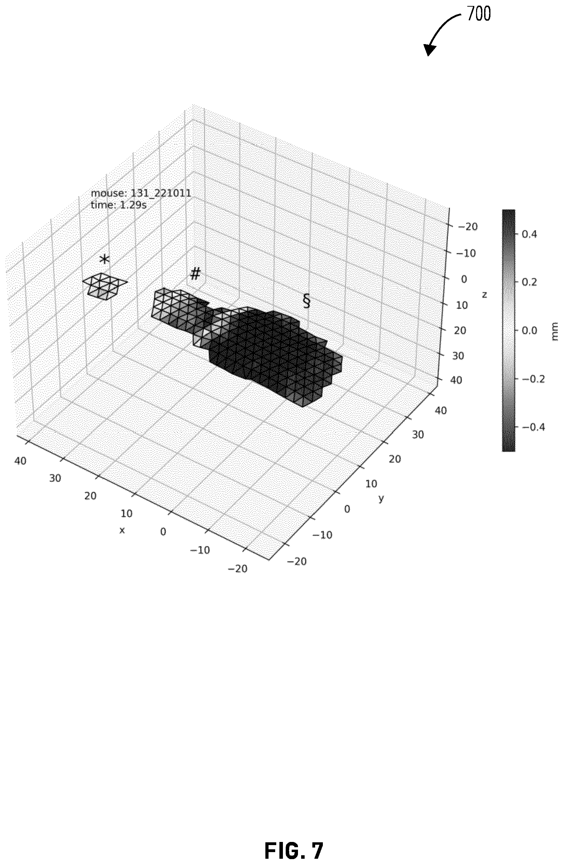

FIG. 7 shows an example of a 3D projection of a generated mesh at one time point. The 3D projection in FIG. 7 represents the chest of the subject at the end of the inhalation phase of breathing. The 3D projection can be colour-coded to show the extent of the movement of the chest relative to a chest position at the end of the exhalation phase.

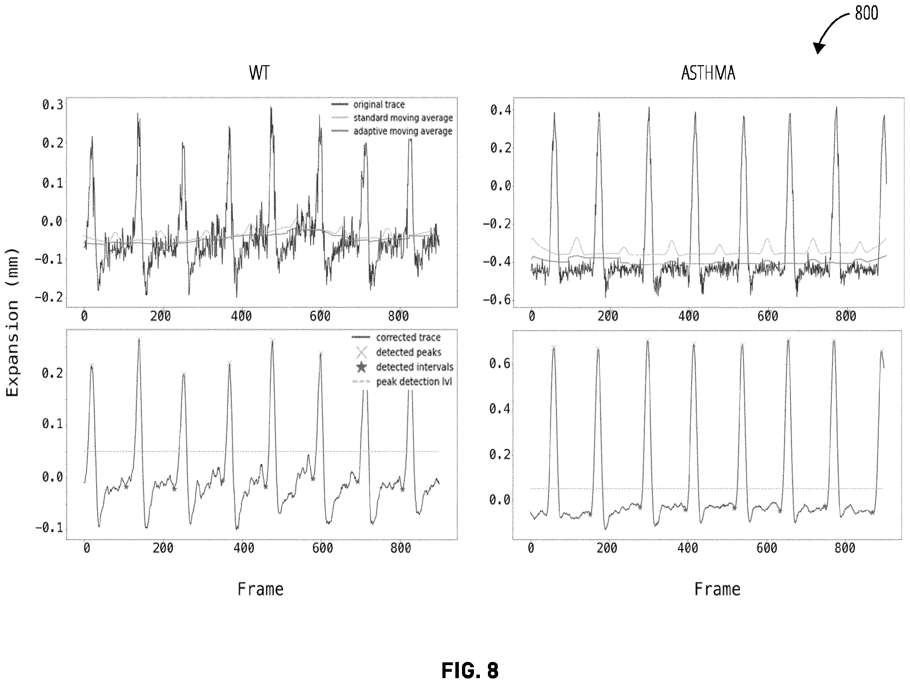

FIG. 8 shows a series of graphs representing examples of the movement over time for an optical marker in the projection pattern. For each point of the generated mesh, the movement over time of the point can be extracted. The result is a raw curve containing multiple signals which change according to the breathing motion of the subject. The raw curves are shown in the top row of graphs.

Adapted moving average filtering can be applied to extract the baseline of the curve and reduce the noise. This results in the curves in the bottom row of graphs. In these post-processed curves, end-inspiration (red asterisk) and end-expiration time points (blue crosses) can clearly be identified. These curves are significant for the diagnosis and staging of lung disease. This is shown by the characteristic differences in the graphs of an asthmatic mouse (right column) compared to a healthy mouse (left column).

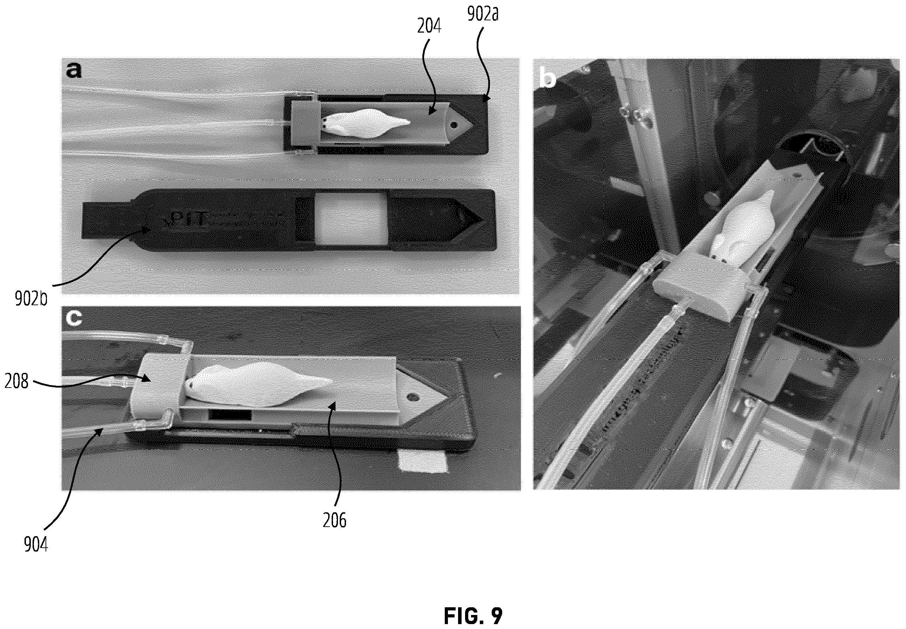

FIG. 9 shows an example of a first mounting structure 902a, a second mounting structure 902b, and a holding means 204. The holding means 204 comprises a support structure 206 which supports the body of the subject (in this case, a replica mouse) and an anaesthesia mask 208 to supply anaesthesia to the subject using anaesthesia tubes 904. In image a) the holding means 204 is shown mounted in a mounting structure 902a,

In image b) the holding means 204 is mounted in the larger mounting structure 902b. The mounting structure 902b is then mounted in an in-vivo computerised tomography (CT) apparatus.

In image c) the holding means 204 is mounted in the smaller mounting structure 902a as part of the apparatus 200.

The holding means 204 may be printed in resin and include connectors for anaesthesia supply and exhaust. The connectors may be a part of the anaesthesia mask 208 and connect to anaesthesia tubes 904. The section of the holding means 204 directly under the chest region of the subject may be modified to a hollowed out half pipe with constant thickness across the entire region. This results in improved CT imaging features.

The mounting structures 902a, 902b may remain in the either the CT device or in the optical setup 200. As the anaesthesia tubes 904 are attached to the holding means 204 itself, the holding means 204 can be moved without changing the status of the mouse. That is, the mouse can be constantly supplied with anaesthesia as the mouse is moved between the CT device and the apparatus 200. Since the mouse is not disturbed during the transportation between the CT device and the apparatus 200 and the mounting structures 902a, 902b are fixed in place, additional markers for image registration are not needed.

**Validation of the OLF derived parameters by the established in vivo $\mu$CT and XLF**

**Animal models and ethics**

Four week old SKH-1 hairless immune-competent female mice were purchased (Charles River) and housed in a regular 12-hour dark:light cycle, at 22°C, with laboratory chow (SAFE, Augy, France) and tap water ad libitum.

**Severe acute allergic asthma (SAA)**

Six female animals were induced with 'severe' acute allergic asthma (SAA) as previously described (Nabe, Takeshi, et al. "Induction of a late asthmatic response associated with airway inflammation in mice." European journal of pharmacology 521.1-3 (2005): 144-155.). Briefly, at 8 weeks (day 0) the animals were immunised with an i.p. injection of 50 $\mu$g OVA (EndoGrade Ovalbumin, Lionex), 0.5% AlOH adjuvant (Alhydrogel 2%, InvivoGen) diluted in 1x PBS (Gibco), 200 $\mu$L total volume and intranasally with 50 $\mu$g OVA in 25 $\mu$L $1\times$ PBS. The immunisation was repeated at day 14. The animals were challenged intranasally with 250 $\mu$g OVA in 25 $\mu$L PBS total volume on days 28, 29, 30 and 33. The mice were imaged at the 'acute' state 48 h after the last challenge. They were then sacrificed, bronchoalveolar lavage (BAL) was performed and the lung was extracted for further analysis.

**Pulmonary fibrosis (PF)**

Six mice received a single 1.5 mg/kg (N=3) or 1 mg/kg (N=3) intratracheal instillation of bleomycin. The animals were weighed daily and were sacrificed early if they displayed signs of dyspnoea in resting state or weight loss of over 20% from the beginning of the experiment. The surviving mice remained in the experiment till day 21 post bleomycin instillation and sacrificed after the final imaging step.

XLF was performed as previously described in Dullin, Christian, et al. "X-Ray based Lung Function measurement-a sensitive technique to quantify lung function in allergic airway inflammation mouse models." Scientific Reports 6.1 (2016): 36297.
Mice were anaesthetised with 2% isoflurane, airflow of 1 L/min of 50:50 mix of air and oxygen. The mouse was then positioned on its back in the mouse holder. The anaesthesia was titrated to maintain the respiratory frequency of 0.7~Hz. This frequency was chosen due to the limiting acquisition frame rate (30-Hz) of the X-ray detector. Planar cinematic x-ray movies were acquired with the following parameters: field of view of 20x20 mm$^2$ 60 $\mu$A, 90 kV, 1024 frames (QuantumFX, Perkin Elmer, Waltham, MA, USA). The acquired data was analysed using custom XLF analysis software (https://gitlab.com/heimdall32/xLFinal).

**Retrospectively gated in-vivo micro computed tomography ($\mu$CT)**

$\mu$CT was performed using 2 rotations acquiring 7920 projections with a field of view of 40x40 mm$^2$, at 200 $\mu$A, 90 kV for 4.5 minutes. Subsequently retrospective gating was performed; the projections were sorted and reconstructed separately yielding 3D reconstruction of end 'inhaled' and 'exhaled' states. The volumes in each reconstructed state were calculated using a region growing algorithm. Each reconstruction was polygonised to create a surface mesh of the chest. The meshes were compared by computing signed euclidean distances from the reference mesh (exhaled state) to the measured mesh (inhaled state) in the same way as with the OLF data using Mesh Lab (V. 2022.2).

### Optical lung function (OLF)

The OLF system was calibrated using a physical checkerboard pattern 8x9 squares with a 5mm side in at least 30 poses. The mouse was anaesthetised to maintain the respiratory frequency at 1 Hz and placed on the same mouse holder used in $\mu$CT and XLF (or moved together with the holder without repositioning if a previous $\mu$CT scan was performed). A dot pattern was projected onto the mouse. Each dot was encoded in a triple binary encoding sequence. The breathing was recorded unrestrained for 10-15-seconds using 2 cameras with a matrix size of 600x400 px and a firing rate of 100 FPS (a2A 1920 160um, Basler) and a projector (LightCrafter 4500, Texas Instruments) using a static dot pattern. The pattern was projected onto a plane of a size of 21x13.5 cm$^2$ with circular markers measuring 1.5 mm in diameter and spaced 3 mm apart (measured in the projection).

### Results

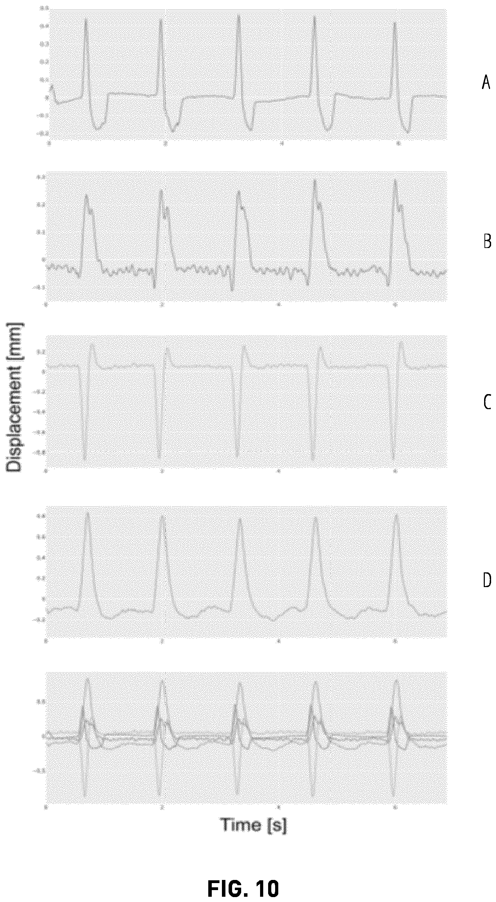

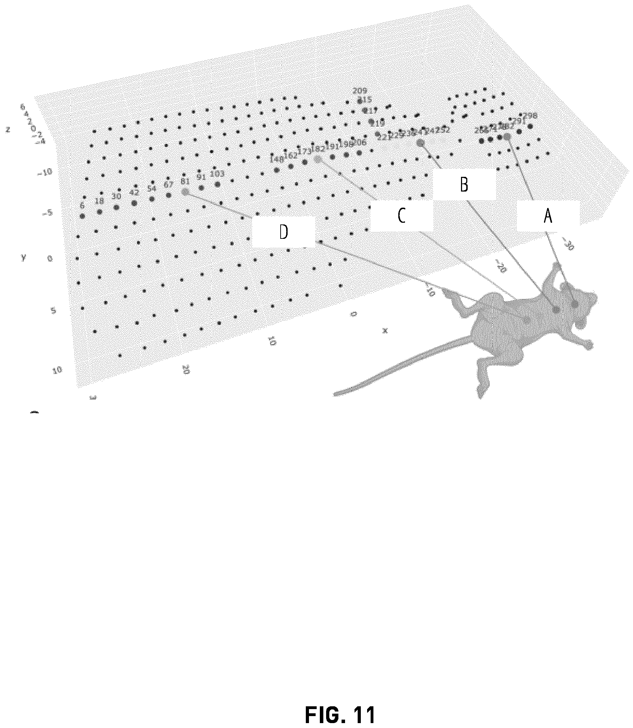

FIG. 10 shows the surface displacement in different anatomical regions of a healthy (CN) mouse, with the corresponding 3D anatomical location shown in Figure 11. OLF demonstrates that the displacement of the chest surface varies with the anatomical location of the measurement point since different anatomical regions show different curve modulations.

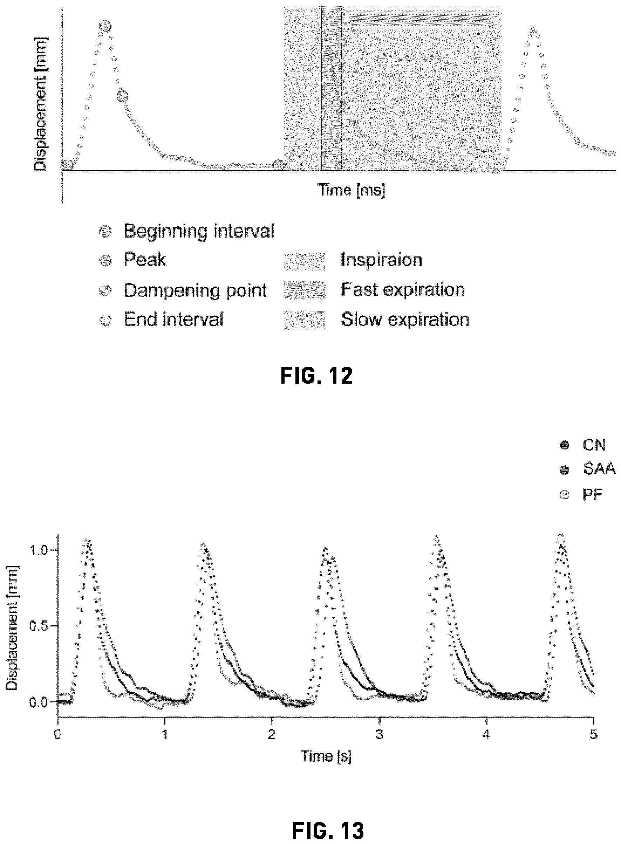

FIG. 12 shows the separation of a breathing event into three phases: inspiration (I), fast expiration (FE), and slow expiration (SE). The displacement in the upper abdominal region (region "A" in Figures 10 and 11) was used for further analysis, with five points selected in the region. The displacement curve from each point was scaled according to the mean maximum and minimum displacements of the curve. It was then separated into individual breathing events. For each "expiration" segment of the breathing event, a double Gaussian function was fitted. The three events with the highest coefficient of determination (greater than 0.98) were analysed further. Next, a dampening point in the expiration curve was derived. The dampening point was defined as the minimum in the third derivative. Each breathing event was then separated into the three phases: inspiration (I) - from the beginning of the breathing event to the peak, fast expiration (FE) - from the peak to the dampening point, and slow expiration (SE) - from the dampening point to the end of the breathing event.

FIG. 13 shows an overlaid example of abdominal markers for a CN, SAA and PF mouse. The shape of the breathing events for each type of mouse is different. The following parameters were calculated: a) for the inspiration segment - inspiration time, slope of inspiration , area under the segment (AuC), b) for the FE - FE time, slope of FE, AuC, c) SE - SE time, SE half life (T1/2, from exponential decay fit), AuC, d) additionally the width of the peak above 30% was quantified. The calculated parameters were first averaged across the three breathing selected breathing events and then the five selected markers for each animal.

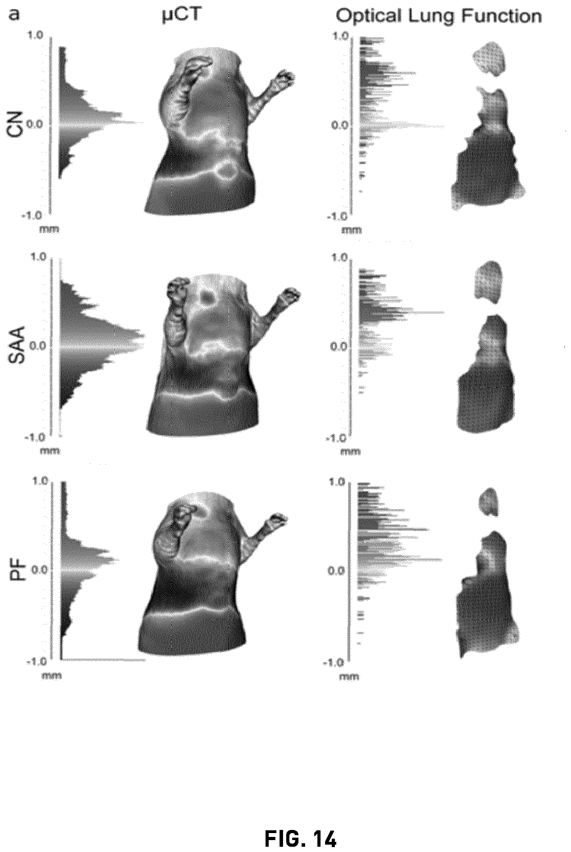

FIG. 14 shows a comparison of the displacement meshes derived by $\mu$CT and OLF. The expansion from the reference exhaled state is denoted above zero and the withdrawal from the reference exhaled state below zero. FIG. 14 shows the corresponding signed distance meshes between end expiration and end inspiration from both the $\mu$CT and the OLF reconstructions. Positive displacement from the exhaled state is the expansion of the surface (red) while negative displacement from the exhaled state is the withdrawal (blue). As shown by the distance histograms the range of surface movement calculated in $\mu$CT is between -1.0 and 1.0 mm, which is in agreement with the surface movement extracted from the OLF data. However, it has to be noted that the OLF method shows one selected breathing event, while the $\mu$CT presents a averaged mix of multiple breathing events.

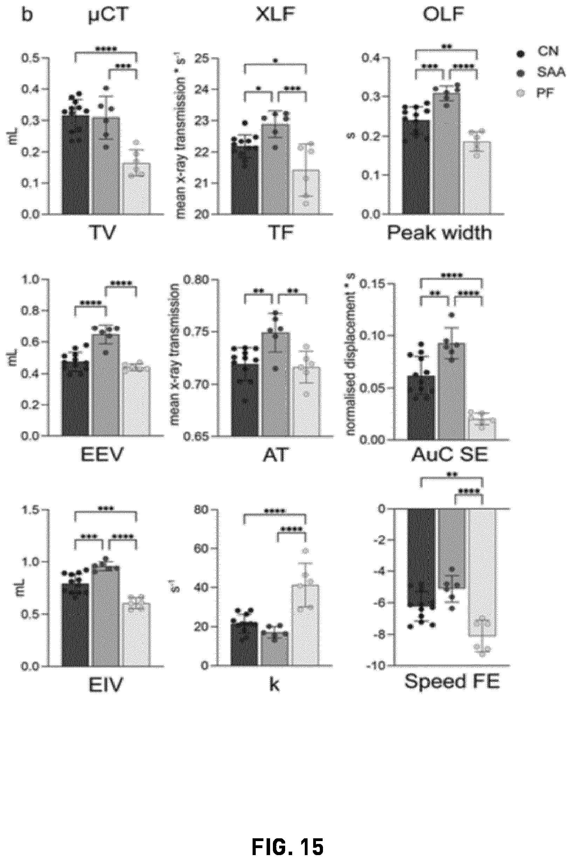

FIG. 15 shows volumes derived from the retrospectively gated $\mu$CT : tidal volume (TV), end expiratory volume (EEV), end inspiratory volume (EIV). Functional parameters derived by XLF : tidal flow (TF), air trapping (AT), expiration constant (k). Displacement parameters derived by OLF: peak width (time for the displacement above 30% of the maximum value), area under the curve for slow expiration (AuC SE), slope of fast expiration (Speed FE). Statistical significance determined by the one-way ANOVA test with Turkey's multiple comparisons: * $p < 0.00332$, ** $p < 0.0021$, *** $p < 0.0002$, **** $p < 0.0001$.

FIG. 15 demonstrates that the OLF functional parameters were validated by the established in vivo XLF and $\mu$CT measurement methods. The parameters are summarized in Table 2. SAA mice showed a comparable TV to CN, with increased an EEV and EIV. In contrast, the PF mice displayed a decreased TV and EIV with an EEV comparable to CN. Planar cinematic x-ray-based lung function (XLF) derived parameters showed an increased TF for the SAA mice and a decreased TF for the PF mice indicating an on average more aerated and less aerated lung respectively, which is consistent with the EIV measurements. In agreement with the EEV, the calculated AT levels were elevated in the SAA mouse model. In contrast, the AT level in the PF mouse model was comparable to the AT level of CN. Finally the k parameter appeared significantly higher for the PF mice compared to CN. The peak width parameter derived from OLF measurement, demonstrated significantly higher values in the SAA mice compared to CN mice. This was consistent with the TF in XLF and EIV in $\mu$CT, suggesting a bigger inspiration effort. Furthermore, the AuC of slow expiration in OLF was significantly larger in the SAA animals, which was consistent with the increased AT measured by XLF and increased EEV measured by $\mu$CT indicating that the expiration phase took longer to return to baseline. Taken together these results support the expected obstructive pathology allergic asthma development. The peak width (OLF) of PF mice was significantly shorter compared to CN, indicating a shortened breathing event. This is supported by the reduced EIV and TF derived by $\mu$CT and XLF imaging. PF animals also displayed a highly reduced AuC for SE (OLF) as well as the increased slope of FE indicating an increase in the elastic recoil of the lung during expiration. These results were consistent with a dramatically high expiration constant k (XLF) and reduced TV ($\mu$CT) observed in the PF mice. These results present a classical pattern of restrictive pulmonary disease.

**Table 2.** Summary of lung function parameters

| Parameter | Technique | Description |
|---|---|---|
| EIV | $\mu$CT | End inspiratory volume |
| EEV | | End expiratory volume |
| TV | | Difference between EIV and EEV |
| TF | XLF | Average normalised x-ray transmission per second between the beginning of the breathing interval and the level function |
| AT | | Average baseline of the normalised x-ray transmission at the end of the breathing event |
| k | | Expiration constant derived from the fitting of an exponential decay function to the expiration segment |
| Peak width | OLF | Time for displacement above 30% of the maximum value |
| AuC SE | | Integrated area of the slow expiration segment |
| Speed FE | | Slope of the linear function fitted to the fast expiration segment |

In order to validate the OLF findings in restrictive and obstructive disease patterns, OLF was correlated with established ex vivo measurement methods of SR$\mu$CT, histology and BAL. Histological evaluation showed clear ongoing inflammatory processes in SAA lungs with thickening of the bronchial walls and immune cell accumulation in the perivascular region. SAA lungs showered an increase in mucus producing cells lining the bronchial walls. The lungs from the PF mice displayed closure of the alveolar air spaces due to fibrotic remodelling due to increased collagen fiber deposition in the lung parenchyma. Additionally PF lungs displayed a diffused enrichment in immune cells present in the consolidated regions. Compared to CN, the inflammation score was higher in SAA and PF, with SAA also having a significantly higher score than PF. The mucus score was significantly higher in the SAA model compared to both the CN and the PF model. The increase in mucus producing cells, typically leading to mucus plugging, was consistent with the increased inspiratory and expiratory efforts calculated by OLF. In contrast to the inflammation score no elevated level of mucus production was found in the PF model. However, the PF model displayed an increase in collagen deposition as evidenced by an elevated modified Ashcroft score indicating progressive fibrotic remodelling in agreement with the observed change in elastic properties calculated by OLF. Overall, the histological evaluation showed the typical features of both SAA and PF and the absence of them in the CN in all specimens, confirming the successful induction of each of the lung disease models.

Phase contrast SR$\mu$CT is an established method for 3D structural quantification of standard formalin fixed paraffin embedded (FFPE) tissue without the need of sectioning. To evaluate the lungs from the different disease models at approximately the same anatomical positions in the lung parenchyma 8 to 10 3D ROIs ($100 \times 100 \times 100$ px) were manually placed. An intensity threshold was manually adjusted to define the interface between lung tissue and the paraffin filled air spaces. The tissue volume ratio per cube, and tissue surface ratio were calculated. The tissue volume ratio showed a significant increase in SAA and even more in PF lungs. The surface area ratio was strongly reduced in lung of PF mice compared to CN which is also supported by the fibrotic remodelling found in the histological analysis. Combined, these results suggest a reduction in alveolar space which in turn indicates impaired gas exchange. No significantly reduced surface area ratio was found in the lungs of SAA compared to CN. This could be explained by the fact that a strong accumulation of eosinophiles was found primarily close to larger bronchi and vessels, whereas the lung parenchyma did not appear to be affected.

In order to evaluate the sensitivity of OLF in comparison to the other applied imaging and analysis methods the biological effect size was calculated. The maximum effect size over all parameters of one method was computed for a group-to-group comparison. OLF was found to outperform XLF, reaching comparable effect size values of $\mu$CT. However, OLF does not use ionizing radiation and a more shallow anesthesia. SR$\mu$CT shows a comparably small effect size, especially for CN vs. SAA and SAA vs. PF. Histology, due to its high specificity, displays the highest discriminatory effect. However SR$\mu$CT and histology can only be performed ex vivo.

Since OLF generates a complete new set of functional parameters, a correlation analysis was performed to understand which features are correlated. It was found that EIV is strongly positively correlated to all OLF parameters (> 0.77), while the expiration constant of XLF is strongly negatively correlated with most of the OLF parameters (< -0. 53). In terms of histological validation, OLF shows a negative correlation to the Ashcroft score (< -0.61) and a positive correlation to the mucus score (> 0.53). The inflammation score was not highly correlated to OLF parameters (< 0.53), which is plausible given the fact that it measures the accumulation of immune cells which usually occurs close to the bigger bronchi and vessels and thus does not influence the breathing as much as mucus plugging or strong fibrotic remodelling do. All three measures of OLF showed a strong internal correlation (> 0.7), indicating that they are in the same way affected by the two different mouse models.

**Uses and diagnostic methods**

The method described herein can be used for diagnosing or monitoring any disease or disorder associated with the lung functionality in subjects in need thereof. The method allows to diagnose whether a subject suffers from a certain disease or disorder, it allows to monitor the progress of a disease or disorder in a subject, and it allows to evaluate treatment regimens in a subject that is treated.

According to one aspect the method according to the invention can be used for diagnosing a disease or disorder, for monitoring a disease or disorder, or for assessing a treatment regimen in a subject.

According to another aspect of the invention a method for diagnosing a disease or disorder, for monitoring a disease or disorder, or for assessing a treatment regimen in a subject, is provided, the method comprising:

projecting, by a projector, a projection pattern comprising a number of optical markers onto a chest of the subject;
capturing, by a stereo-camera system, a series of images of the projection pattern from at least two angles as the subject breathes, such that each optical marker from the projection pattern is imaged from both of the at least two angles at the same time;
tracking, by a processor, a movement over time of the projection pattern based on the captured series of images by tracking a movement over time of each of the optical markers in the captured series of images; and

determining, by the processor, at least one parameter indicative of the chest function of the subject based on a result of the tracking.

Herein the terms "diseases", "disorders", and "conditions" are used interchangeably and refer to a disorder of structure or function in a human or animal, especially one that produces specific symptoms or that affects a specific location and can be a direct result of physical injury. In one example the disease or disorder is associated with an impairment of the lung function. As used herein, the phrase "a disorder or disorder associated with an impairment of the lung function" refers to but is not limited to any disease or disorder resulting directly or indirectly from and/or completely or partially from an impaired lung function.
In one example the disease or disorder is a lung disease. The lung disease can be an airway disease, a lung tissue disease, or a lung circulation disease. In one example the disease is asthma or pulmonary fibrosis (PF). In one example the disease or disorder is severe acute allergic asthma (SAA). In one example the lung disease is selected from the group consisting of asthma, collapse of part or all of the lung (pneumothorax or atelectasis), swelling and inflammation in the main passages (bronchial tubes) that carry air to the lungs (bronchitis), COPD, lung cancer, lung infection (pneumonia), abnormal buildup of fluid in the lungs (pulmonary edema), and blocked lung artery (pulmonary embolus).

The invention was explained above with reference to a specific embodiment. Some modifications are possible within the scope of the invention. In the above embodiment the sequence of images is projected onto the subject only once. It can make sense to repeatedly project the sequence of images onto the subject during the encoding phase, particularly if the position of the markers is strongly changing in the detected images due to the movement of the subject. A repeatedly projection of the sequence of the images allows a safe identification of the markers, even if there are significant changes of the position of the markers in the detected images occurs.

In the case of a very strong variation of the position of the markers in the detected images it can be useful to encode the markers permanently or repeatedly during the dynamic phase in parallel to the detection of the movement of the surface of the subject. In such a case the encoding phase can be even eliminated.

Definitions for the various aspects, examples, embodiments, modifications of the of the invention might be provided elsewhere herein and equally apply here. Definitions, embodiments, examples etc. herein for one aspect of the invention equally apply for all the other aspects of the invention. Unless it is apparent from the context, each of the embodiments and examples listed herein can be applied for use in any of the aspects of the invention.

**Claims**

1. A method for determining a chest function of a subject, the method comprising:

   projecting, by a projector, a projection pattern comprising a number of optical markers onto a chest of the subject;
   capturing, by a stereo-camera system, a series of images of the projection pattern from at least two angles as the subject breathes, such that each optical marker from the projection pattern is imaged from both of the at least two angles at the same time;
   tracking, by a processor, a movement over time of the projection pattern based on the captured series of images by tracking a movement over time of each of the optical markers in the captured series of images; and
   determining, by the processor, at least one parameter indicative of the chest function of the subject based on a result of the tracking.

2. The method of claim 1, further comprising generating a unique identifier, ID, for each optical marker.

3. The method of claim 2, wherein generating the unique ID for each optical marker further comprises:

   projecting, by the projector, a series of optical marker arrangements onto the chest of the subject,
   wherein each arrangement comprises one or more of the optical markers from the projection pattern, and
   wherein each optical marker is present in at least one arrangement from the series of arrangements;
   detecting, by the stereo-camera system, the one or more optical markers in each of the projected arrangements from the at least two angles; and
   generating, by the processor, the ID for each optical marker based on the at least one arrangement in which the optical marker is detected.

4. The method of any one of claims 1 to 3, wherein tracking the movement over time of the projection pattern comprises:

   generating one or more of a two-dimensional, 2D, projection and a three-dimensional, 3D, projection of the projection pattern at each of a series of time points;
   extracting, from the one or more generated projections, a movement over time of each of the optical markers of the projection pattern.

5. The method of any one of claims 1 to 4, wherein determining the at least one parameter indicative of the chest function of the subject comprises:

   reconstructing a four-dimensional, 4D, motion of the chest based on a result of the tracking of the projection pattern; and
   extracting, from the reconstructed motion of the chest, the at least one parameter.

6. The method of any one of claims 1 to 5, wherein the at least one parameter comprises one or more of a general temporal parameter, a local temporal parameter, a moving speed of a portion of the chest, a diaphragm motion parameter, a tidal volume indicator, or a parameter indicative of a lung function of the subject.

7. The method of any one of claims 1 to 6, wherein the sampling rate is at least 10 frames per second.

8. The method of any one of claims 1 to 7, wherein the sampling rate is less than 200 frames per second.

9. The method of any one of claims 1 to 8, wherein one or more of a size of each of the optical markers, a spacing between each of the optical markers, and a number of the optical markers is determined based on a size of a surface area the of chest of the subject.

10. The method of any one of claims 1 to 9, wherein the optical markers are unequally distributed across the chest particularly such that regions of the chest which have a greater range of movement while the subject is breathing and/or are more indicative of the chest function of the subject have a greater density of optical markers.

11. The method of any one of claims 1 to 10, wherein the optical markers have a luminance of at least twice a background luminance.

12. An apparatus for determining a chest function of a subject, the apparatus comprising:

a projector configured to project a projection pattern of optical markers onto a chest of the subject;
stereo-camera system configured to capture a series of images of the projection pattern from at least two angles as the subject breathes, such that each optical marker from the projection pattern is imaged from both of the at least two angles at the same time; and
a processor, wherein the processor is configured to:

track a movement over time of each of the optical markers based on the captured series of images; and
determine at least one parameter relating to the chest function of the subject based on a result of the tracking.

13. The apparatus of claim 12, wherein the processor is further configured to:

control the projector to project the projection pattern of optical markers onto the chest of the subject; and
control the stereo-camera system to capture the series of images of the projection pattern as the subject breathes.

14. The apparatus of claim 12 or 13, further comprising a mounting structure for mounting to a holding means for holding the subject.

15. The apparatus of claim 14, further comprising the holding means for holding the subject, the holding means comprising:

a support structure for supporting a body of the subject; and
an anaesthesia mask for providing anaesthesia to the subject.

100

102

104

106

**FIG. 1**

200

102

104

106

202

204

206

208

**FIG. 2**

300

302

PROJECT A PROJECTION PATTERN
ONTO A SUBJECT

304

CAPTURE A SERIES OF IMAGES

306

TRACK A MOVEMENT OF THE
PROJECTION PATTERN

308

DETERMINE A PARAMETER
INDICATIVE OF THE CHEST
FUNCTION

**FIG. 3**

FIG. 4

**FIG. 5**

*      #      §

600

**FIG. 6**

**FIG. 7**

**FIG. 8**

EP 4 603 014 A1

**FIG. 9**

FIG. 10

FIG. 11

**FIG. 12**

- ○ Beginning interval
- ○ Peak                    ▨ Inspiraion
- ○ Dampening point    ▨ Fast expiration
- ○ End interval           ▨ Slow expiration

**FIG. 13**

**FIG. 14**

FIG. 15

Europäisches
Patentamt

European
Patent Office

Office européen
des brevets

# EUROPEAN SEARCH REPORT

**Application Number**

EP 24 15 7451

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 311 740 A1 (PNEUMACARE LTD [GB]) 25 April 2018 (2018-04-25) * figure 9 * * paragraph [0031] - paragraph [0033] * * paragraphs [0063], [0070], [0080] * | 1-15 | INV. A61B5/113 A61B5/11 |
| X | US 2014/140592 A1 (LASENBY JOAN [GB] ET AL) 22 May 2014 (2014-05-22) * paragraphs [0056], [0070] - [0072], [0083]; claim 14; figures 2, 3 * | 1,2,4-8, 12,13 | |
| A | | 3,11 | |
| A | US 2015/187082 A1 (BERNAL EDGAR A [US] ET AL) 2 July 2015 (2015-07-02) * paragraph [0026] * | 3,9,10 | |
| A | VAN DER JEUGHT SAM ET AL: "Real-time structured light profilometry: a review", OPTICS AND LASERS IN ENGINEERING, ELSEVIER, AMSTERDAM, NL, vol. 87, 11 February 2016 (2016-02-11), pages 18-31, XP029721400, ISSN: 0143-8166, DOI: 10.1016/J.OPTLASENG.2016.01.011 * figure 2 * | 3 | |
| A | EP 2 975 834 A1 (XENOGEN CORP [US]) 20 January 2016 (2016-01-20) * paragraphs [0006], [0055] * | 14,15 | TECHNICAL FIELDS SEARCHED (IPC) A61B |

-/--

The present search report has been drawn up for all claims

| Place of search | Date of completion of the search | Examiner |
|---|---|---|
| The Hague | 18 April 2024 | Athanasiadis, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

**EUROPEAN SEARCH REPORT**

Europäisches
Patentamt
European
Patent Office
Office européen
des brevets

Application Number

EP 24 15 7451

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE BOER WILLEM ET AL: "SLP: A Zero-Contact Non-Invasive Method for Pulmonary Function Testing", PROCEEDINGS OF THE BRITISH MACHINE VISION CONFERENCE; ABERYSTWYTH., 1 January 2010 (2010-01-01), pages 85.1-85.12, XP093151520, London, GB DOI: 10.5244/C.24.85 ISBN: 978-1-901725-40-7 Retrieved from the Internet: URL:https://www.researchgate.net/profile/Willem-De-Boer/publication/221259606_SLP_A_Zero-Contact_Non-Invasive_Method_for_Pulmonary_Function_Testing/links/55c8ab8d08aea2d9bdc91721/SLP-A-Zero-Contact-Non-Invasive-Method-for-Pulmonary-Function-Testing.pdf > [retrieved on 2024-04-15] * page 3; figure 1 * | 1,2,4-6, 12 | |
| | ----- | | **TECHNICAL FIELDS SEARCHED** (IPC) |

The present search report has been drawn up for all claims

| Place of search | Date of completion of the search | Examiner |
|---|---|---|
| The Hague | 18 April 2024 | Athanasiadis, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 7451

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2024

| Patent document cited in search report | | Publication date | Patent family member(s) | | Publication date |
|---|---|---|---|---|---|
| EP 3311740 | A1 | 25-04-2018 | CN | 104902816 A | 09-09-2015 |
| | | | CN | 107468256 A | 15-12-2017 |
| | | | DK | 2925227 T3 | 25-06-2018 |
| | | | EP | 2925227 A1 | 07-10-2015 |
| | | | EP | 3311740 A1 | 25-04-2018 |
| | | | ES | 2673117 T3 | 19-06-2018 |
| | | | GB | 2513210 A | 22-10-2014 |
| | | | GB | 2532883 A | 01-06-2016 |
| | | | US | 2015305657 A1 | 29-10-2015 |
| | | | US | 2018360351 A1 | 20-12-2018 |
| | | | WO | 2014083337 A1 | 05-06-2014 |
| US 2014140592 | A1 | 22-05-2014 | AU | 2009324342 A1 | 07-07-2011 |
| | | | CA | 2746483 A1 | 17-06-2010 |
| | | | CN | 102301202 A | 28-12-2011 |
| | | | DK | 2384420 T3 | 27-06-2016 |
| | | | EP | 2384420 A2 | 09-11-2011 |
| | | | JP | 2012511707 A | 24-05-2012 |
| | | | KR | 20110105789 A | 27-09-2011 |
| | | | US | 2013002832 A1 | 03-01-2013 |
| | | | US | 2014140592 A1 | 22-05-2014 |
| | | | WO | 2010066824 A2 | 17-06-2010 |
| US 2015187082 | A1 | 02-07-2015 | US | 2015187082 A1 | 02-07-2015 |
| | | | US | 2016086341 A1 | 24-03-2016 |
| EP 2975834 | A1 | 20-01-2016 | AU | 2006243966 A1 | 16-11-2006 |
| | | | CA | 2605346 A1 | 16-11-2006 |
| | | | CN | 101310515 A | 19-11-2008 |
| | | | EP | 1889470 A2 | 20-02-2008 |
| | | | EP | 2975834 A1 | 20-01-2016 |
| | | | JP | 2008541092 A | 20-11-2008 |
| | | | US | 2005237423 A1 | 27-10-2005 |
| | | | US | 2008079802 A1 | 03-04-2008 |
| | | | WO | 2006122229 A2 | 16-11-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NABE** ; **TAKESHI et al.** Induction of a late asthmatic response associated with airway inflammation in mice.. *European journal of pharmacology*, 2005, vol. 521 (1-3), 144-155

- **DULLIN** ; **CHRISTIAN et al.** X-Ray based Lung Function measurement-a sensitive technique to quantify lung function in allergic airway inflammation mouse models.. *Scientific Reports*, 2016, vol. 6.1, 36297